# EUROPEAN PATENT APPLICATION

(11) **EP 1 616 560 A1**
(43) Date of publication of application: **18.01.2006**
(21) Application number: 03816023.0
(22) Date of filing: 11.08.2003
(51) Int. Cl.: A61K 9/10, A61K 35/12

(54) **FATTY EMULSION INJECTION OF SEAL OIL, METHOD FOR PREPARATION AND THE USE IN MANUFACTURING INTRAVENOUS INJECTION**

(30) Priority: 26.02.2003 CN 03105287
(71) Applicant: Liu, Wei, Beijing 100026 (CN); Liu, Junxiang, Beijing 100026 (CN); He, Weijian, Beijing 100026 (CN); Liu, Yun, Beijing 100026 (CN); Yan, Xijun, Ianjin 300402 (CN)
(72) Inventor: LIU, Wei, Bldg 8, Unit 4, Room 101 Beijing 10002 (CN); LIU, Junxiang, Bldg 8, Unit 4, Room 101 Beijing 10002 (CN); HE, Weijian, Bldg 8, Unit 4, Room 101 Beijing 10002 (CN); LIU, Yun, Bldg 8, Unit 4, Room 101 Beijing 10002 (CN); YAN, Xijun, Xin Uuan Bai Road Ianjin 300402 (CN); XU, Kangsen, Bldg 8, Unit 4, Room 101 Beijing 10002 (CN)
(74) Representative: Fyles, Julie Marie
(86) International application number: PCT/CN2003/000658
(87) International publication number: WO 2004/075878

(57) **Abstract**

The present invention relates to a seal oil based lipid emulsion injection, and the main ingredients of which contains 190-210 g/l of refined OMEGA3 seal oil, 11-13 g/l of refined lecithin, 24-26 g/l of glycerol for injection, and balance amount of water. The preparation of the seal oil based lipid emulsion injection comprising the steps of stir and dispersion, high-pressure homogenization, vacuum filtration, antisepsis and encapsulation. The OMEGA3 seal oil lipid based emulsion injections have high content of energy, and thus are highly absorbable to human body, and can not only provide human body with caloricity, but also supply the fatty acids necessary for human body, and can enhance body's immunizing ability, reduce the content of cholesterol, adjust the blood concentration and then be used for anti-inflammation, in particular, it is highly useful for a postoperative patient to restore his strength.

## Description

### Field of the Invention

The present invention relates to a seal oil based lipid emulsion injection, preparation thereof and uses thereof for the preparation of intravenous injections. In particular, the present invention relates to the OMEGA 3 seal oil based emulsion injection, preparation thereof and uses thereof for the preparation of intravenous injections.

### Background Art

Lipid is a nutritive factor containing the highest caloricity, each gram of which provides 9.3 kilocalories of caloricity. Most of the energy of human body is stored in the form of lipid. And when a body is in short supply of energy as a result of wounds or suffering from various diseases, the self-stored lipid will be utilized to generate caloricity, meanwhile organism protein (mainly mytolin) will be synthesized into glucose via glyconeogensis, in such way the metabolism balance of human body is able to be maintained.

After the food lipid is normally ingested by human body, it will be emulsificated via bile salt into tiny colloidal particles with diameters no more than 0.5 µm in the intestines, and then be decomposed into lipid acid and glycerolester by esterolysis enzymes, and then be ingested by intestinal wall mucous membrane via diffusion, and then be synthesized into triglyceride within the intestinal wall mucous membrane cell, and then be formulated into chyle in conjunction with parts of proteins and α-phosphoglyceride, and this chyle will flow into the intestinal wall lymphatic vessel, after influxing into the chest vessel, it will flow into the superior vena cava. When a body is not able to take food orally because of wounds or diseases, and thus is in malfunction of lipid ingestion, mainline of these kinds of nutritive factor is in need. To meet this end, it is necessary to formulate the lipid foods into chyle particles with diameters no more than 1 µm, and then inject them into the veins in *vitro* to supply the histiocytes. Lipid emulsion is a man-made chyle liquid which play an important role in meeting the human body's energy demands, and maintaining cells ecology, and also enhancing immunity.

The lipid emulsion provided in the prior arts mostly contain bean oil and carthamus oil as the main ingredients, for example, CN1376460A disclosed a lipid emulsion used for injection and preparation thereof, and the main ingredients of this lipid emulsion are: 270-330 g/l of purified bean oil, 11-13 g/l of refined lecithin, 15-18 g/l anhydrous glycerol, quantum satis of sodium hydroxide, and the remains is injection water. So far, no prior arts both here and abroad have report the use of OMEGA 3 seal oil for the preparation of lipid emulsion injections.

The lipid emulsion of the present invention contains refined OMEGA 3 seal oil as main ingredient. Seal is the popular name of *harp seal*, a seal family animal inhabiting arctic pole and northern Atlantic Ocean. Out of the successful protective measures of the Canadian government, the quantity of seal is increasing at a stable speed of 7% annually. In order to maintain ecology balance between the seals and fish school, it is allowed to hunt a small quantity of seal under the permission of the Canadian government. The product of the present invention is derived from the subcutaneous fat of newly hunted arctic pole seal, And after vacuum evaporation, and subsequent removal of the protein and hydrocarbons and the like contained therein under low temperature, a yellowish transparent liquid refined OMGA3 seal oil is obtained. Seal oil is rich in Ω-3 poly-carbon unsaturate fatty acids, which are necessary to human body and can not be synthesized endosomatically, and so its nutritive value is much higher than that of edible oil. Besides the eicosapentaenoic acid (EPA) and docosahexaenoic acid (DHA), which are generally contained in fish oil, significant amounts of docosapentaenoic acid (DPA) are also found in the OMEGA3 seal oil. In the OMEGA3 seal oil, which are refined and processed under optimized conditions, the content of EPA, DHA and DPA can be up to about 25%. Small quantities of squalene (SQUALENE) are also contained in the OMEGA3 seal oil, but nearly no cholesterol is found therein. Large quantity of Ω-3 fatty acid are contained in the OMEGA3 seal oil, whereas soybean oil and safflower oil mainly contains Ω-6 fatty acids. The emulsion injections prepared from OMEGA3 seal oil are highly absorbable to human body, as the molecular structures highly resemble those of the endogenous fatty acids. In particular, the medical researches in the past two decades have established that the balance between Ω-3 and Ω-6 is of vital to human health, and WHO suggests that the ratio between Ω-3 and Ω-6 should be 1:1. However, as the result of our dietary structure, the ratio of human body's fat remains 1:35, that is to say, Ω-3 fatty acids are too less, while Ω-6 fatty acids are too much, the ratio between them is highly unbalanced, thus, the emulsion injection prepared from OMEGA3 seal oil can provide a better effect than convention lipid based emulsion injections.

The OMEGA3 seal oil lipid based emulsion injections can not only provide human body with caloricity, but also supply the fatty acids necessary for human body, and can enhance body's immunizing ability, reduce the content of cholesterol, adjust the blood concentration and then be used for anti-inflamination, in particular, it is highly useful for a postoperative patient to restore his strength.

### Detailed Description of the Invention

An objective of the present invention is to provide an OMEGA3 seal oil lipid based emulsion injection, whose main ingredient is refined OMEGA3 seal oil, and it is a nutrition emulsion which can not only provide human body with caloricity, but also supply the fatty acids necessary for human body.

In accordance with another aspect of the present invention, there is provided a preparation method of the OMEGA3 seal oil lipid based emulsion injection, which is unique and can be easily operated.

In accordance with another aspect of the present invention, there is provided a use of the OMEGA3 seal oil lipid based emulsion injection in the preparation of intravenous injections.

In one embodiment of the present invention, the OMEGA3 seal oil lipid based emulsion injection comprising:

| | |
|---|---|
| Refined OMEGA3 seal oil | 190-210 g/l |
| Refined lecithin | 11-13 g/l |
| Glycerol for injection | 24-26 g/l |
| Water for injection | add up to 1000 ml |

In another embodiment of the present invention, the OMEGA3 seal oil lipid based emulsion injection comprising:

| | |
|---|---|
| Refined OMEGA3 seal oil | 200 g/l |
| Refined lecithin | 12 g/l |
| Glycerol for injection | 25 g/l |
| Water for injection | add up to 1000 ml |

This product is a white emulsive liquid (i.e. water-encased-oil type), the negative electric charges carried by the emulsion pellets are determined with electrophoresis, the pellets are homogeneously dispersed with diameters no more than 2µm.

In another embodiment of the present invention, the, preparation method of the OMEGA3 seal oil lipid based emulsion injection comprising the following steps:
(1) Stir and dispersion: each ingredient is weighed according the specified ratio, then subject the lecithin into a high speed tissue triturator, after which glycerol for injection and suitable amount of water for injection are added, and stir the mixture into homogenous dispersion under nitrogen atmosphere, then purred into the liquid tank in the two-step homogenizer, and continuously purge in nitrogen.
(2) High-Pressure Homogenization: under the pressure between that above 105 kg/cm² and that below 317 kg/cm² , homogenize the refined OMEGA3 seal oil and phospholipid dispersion to form a crude emulsion, then subject the emulsion under nitrogen flow to circulation homogenization between the two tanks, before the 3rd circulation begins, water for injection is added, after seven circulations, at which time the oil particles sizes are less then 1µm, adjust the pH of the emulsion to 5.5-7 with caution, run out the emulsion.
(3) Vacuum Filtration: cool down the collected emulsion, vacuum filtrated with 4# glass sand funnel under nitrogen flow until no eyewinker remains.
(4) Antisepsis and Encapsulation: transfer the emulsion thus obtained into a bottle, fasten lid, after preheated to 90 °C, again sterilized for 15 minutes under 120 °C, cool down with hot water, expose to room temperature and yield the product.

After check out of items such as particle sizes determination, hemolysis, pyrogen, content determination of oil and glycerol, peroxidation value, acid value, and pH, the product of the present invention is packaged with the specification of 100 ml/bottle * 10 bottles/box, and then is stored in the temperature below 25 °C, and is shake up before use. It is preferred to store the product of the present invention in the refrigerator all years around, and non-low-temperature storage will give rise to the agglomeration of the oil particles. Besides, freezing should strictly prevented, otherwise, the emulsion will be destroyed.

The present invention further provides the use of the OMEGA3 seal oil lipid based emulsion injection in the preparation of intravenous injections. The product of the present invention is a high caloric transfusion used for postoperative and short-term supply, and can supply human body with necessary fatty acids, for example, when it is used in combination with sufficient protein and carbohydrate, will significantly reduce the negative nitrogen balance aroused by the catabolism during the early postoperative phase.

When use for intravenous injection, the dosage of the OMEGA3 seal oil lipid based emulsion injection of the present invention for an adult is no more than 2.5 g lipid injection amount per kilogram body weight every day on average. In each injection, the speed should be controlled within 1 ml per minute in the first 15-30 minutes, and if no deuteropathy arise, it can be increased to 80-100 ml per hour.

The acute toxicity testing of the OMEGA3 seal oil lipid based emulsion injection of the present invention gives results as the following.

Eight dogs with body weight of about 12 kg are tested. When the animal are in normal state and in right sense, a one shot administration is give to them at the speed of 6 g lipid/kg body weight, one week later, pathology slicing examination is carried out to check whether there arise perceivable abnormity or not, and final result show that the product of the present invention is nontoxic.

Clinic examination: 35 adults are injected intravenously with the OMEGA3 seal oil based lipid emulsion of the present invention, after that, the concentration of blood triglyceride and blood total cholesterol remained no change and slightly decreased. And this results conform that the use of the OMEGA3 seal oil based lipid emulsion of the present invention will not give rise to the side effect such as high blood fat.

### Examples

It should be understood that these examples are for illustrative purpose only; therefore, they should not limit the scope of this invention in any way.

### Example 1

200 g/l of refined OMEGA3 seal oil, 12 g/l of refined lecithin, 25 g/l of glycerol for injection, and balance amount of water for injection were mixed, the OMEGA3 seal oil based lipid emulsion injection is prepared in the following steps:
(1) Stir and dispersion: subject the lecithin into a high speed tissue triturator, after which glycerol for injection and suitable amount of water for injection are added, and then stir the mixture into homogenous dispersion under nitrogen atmosphere, then purred into the liquid tank in the two-step homogenizer, and continuously purge in nitrogen.
(2) High-Pressure Homogenization: under the pressure between that above 105 kg/cm² and that below 317 kg/cm², homogenize the refined OMEGA3 seal oil and phospholipid dispersion to form a crude emulsion, then subject the emulsion under nitrogen flow to circulation homogenization between the two tanks, before the 3rd circulation begins, water for injection is added, after seven circulations, at which time the oil particles sizes are less then 1µm, adjust the pH of the emulsion to 6.3 with caution, run out the emulsion.
(3) Vacuum Filtration: cool down the collected emulsion, vacuum filtrated with 4# glass sand funnel under nitrogen flow until no eyewinker remains.
(4) Antisepsis and Encapsulation: transfer the emulsion thus obtained into a bottle, fasten lid, after preheated to 90°C, again sterilized for 15 minutes under 120 °C, cool down with hot water, expose to room temperature and yield the product.

### Example 2

190 g/l of refined OMEGA3 seal oil, 11 g/l of refined lecithin, 24 g/l of glycerol for injection, and balance amount of water for injection were mixed, the OMEGA3 seal oil based lipid emulsion injection is prepared in the following steps:
(1) Stir and dispersion: subject the lecithin into a high speed tissue triturator, after which glycerol for injection and suitable amount of water for injection are added, and then stir the mixture into homogenous dispersion under nitrogen atmosphere, then purred into the liquid tank in the two-step homogenizer, and continuously purge in nitrogen.
(2) High-Pressure Homogenization: under the pressure between that above 105 kg/cm² and that below 317 kg/cm² , homogenize the refined OMEGA3 seal oil and phospholipid dispersion to form a crude emulsion, then subject the emulsion under nitrogen flow to circulation homogenization between the two tanks, before the 3rd circulation begins, water for injection is added, after seven circulations, at which time the oil particles sizes are less then 1µm, adjust the pH of the emulsion to 5.5 with caution, run out the emulsion.
(3) Vacuum Filtration: cool down the collected emulsion, vacuum filtrated with 4# glass sand funnel under nitrogen flow until no eyewinker remains.
(4) Antisepsis and Encapsulation: transfer the emulsion thus obtained into a bottle, fasten lid, after preheated to 90 °C, again sterilized for 15 minutes under 120 °C, cool down with hot water, expose to room temperature and yield the product.

### Example 3

210 g/l of refined OMEGA3 seal oil, 13 g/l of refined lecithin, 26 g/l of glycerol for injection, and balance amount of water for injection were mixed, the OMEGA3 seal oil based lipid emulsion injection is prepared in the following steps:
(1) Stir and dispersion: subject the lecithin into a high speed tissue triturator, after which glycerol for injection and suitable amount of water for injection are added, and then stir the mixture into homogenous dispersion under nitrogen atmosphere, then purred into the liquid tank in the two-step homogenizer, and continuously purge in nitrogen.
(2) High-Pressure Homogenization: under the pressure between that above 105 kg/cm² and that below 317 kg/cm² , homogenize the refined OMEGA3 seal oil and phospholipid dispersion to form a crude emulsion, then subject the emulsion under nitrogen flow to circulation homogenization between the two tanks, before the 3rd circulation begins, water for injection is added, after seven circulations, at which time the oil particles sizes are less then 1µm, adjust the pH of the emulsion to 5.5 with caution, run out the emulsion.
(3) Vacuum Filtration: cool down the collected emulsion, vacuum filtrated with 4# glass sand funnel under nitrogen flow until no eyewinker remains.
(4) Antisepsis and Encapsulation: transfer the emulsion thus obtained into a bottle, fasten lid, after preheated to 90 °C, again sterilized for 15 minutes under 120 °C, cool down with hot water, expose to room temperature and yield the product.

## Claims

1. An OMEGA3 seal oil lipid based emulsion injection comprising:
| | |
|---|---|
| Refined OMEGA3 seal oil | 190-210 g/l |
| Refined lecithin | 11-13 g/l |
| Glycerol for injection | 24-26 g/l |
Balance amount of water

2. An OMEGA3 seal oil lipid based emulsion injection comprising:
| | |
|---|---|
| Refined OMEGA3 seal oil | 200 g/l |
| Refined lecithin | 12 g/l |
| Glycerol for injection | 25 g/l |
Balance amount of water

3. The preparation method of the OMEGA3 seal oil lipid based emulsion injection according to claim 1, comprising the following steps:
(1) Stir and Dispersion: each ingredient is weighed according the specified ratio, and subject the lecithin into a high speed tissue triturator, and then glycerol for injection and suitable amount of water for injection are added, and the mixture is stirred into homogenous dispersion under nitrogen atmosphere, then purred into the liquid tank in the two-step homogenizer, and continuously purge in nitrogen.
(2) High-Pressure Homogenization: under the pressure between that above 105 kg/cm² and that below 317 kg/cm² , homogenize the refined OMEGA3 seal oil and phospholipid dispersion to form a crude emulsion, then subject the emulsion under nitrogen flow to circulation homogenization between the two tanks, before the 3rd circulation begins, water for injection is added, after seven circulations, at which time the oil particles sizes are less then 1µm, adjust the pH of the emulsion to 5.5-7 with caution, run out the emulsion.
(3) Vacuum Filtration: cool down the collected emulsion, vacuum filtrated with 4# glass sand funnel under nitrogen flow until no eyewinker remains.
(4) Antisepsis and Encapsulation: transfer the emulsion thus obtained into a bottle, fasten lid, after preheated to 90 °C, again sterilized for 15 minutes under 120 °C, cool down with hot water, expose to room temperature and yield the product.

4. The use of the OMEGA3 seal oil lipid based emulsion injection according to claim 1 in the preparation of intravenous injection.
